(19) Europäisches Patentamt
European Patent Office
Office européen des brevets

(11) **EP 3 627 093 B1**

(12) **EUROPEAN PATENT SPECIFICATION**

(45) Date of publication and mention
of the grant of the patent:
**09.11.2022 Bulletin 2022/45**

(21) Application number: **18461611.8**

(22) Date of filing: **21.09.2018**

(51) International Patent Classification (IPC):
**G01B 9/02091** *(2022.01)*   **G01B 9/02** *(2022.01)*
**G01B 9/02001** *(2022.01)*   **A61B 3/10** *(2006.01)*
**G01B 9/02055** *(2022.01)*   **G01B 9/02004** *(2022.01)*

(52) Cooperative Patent Classification (CPC):
**G01B 9/02044; A61B 3/102; G01B 9/02004;
G01B 9/0201; G01B 9/02038; G01B 9/02062;
G01B 9/02082; G01B 9/02083; G01B 9/02091;**
G01B 2290/65

(54) **APPARATUS FOR PARALLEL FOURIER DOMAIN OPTICAL COHERENCE TOMOGRAPHY IMAGING AND IMAGING METHOD USING PARALLEL FOURIER DOMAIN OPTICAL COHERENCE TOMOGRAPHY**

VORRICHTUNG ZUR PARALLELEN OPTISCHEN KOHÄRENZTOMOGRAPHIE-BILDGEBUNG IM FOURIER-BEREICH UND BILDGEBUNGSVERFAHREN MIT VERWENDUNG VON PARALLELER OPTISCHER KOHÄRENZTOMOGRAPHIE IM FOURIER-BEREICH

APPAREIL D'IMAGERIE DE TOMOGRAPHIE PAR COHÉRENCE OPTIQUE EN PARALLÈLE DANS LE DOMAINE DE FOURIER ET PROCÉDÉ D'IMAGERIE PAR TOMOGRAPHIE À COHÉRENCE OPTIQUE EN PARALLÈLE DANS LE DOMAINE DE FOURIER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.03.2020 Bulletin 2020/13**

(73) Proprietor: **Instytut Chemii Fizycznej Polskiej Akademii Nauk**
**01-224 Warszawa (PL)**

(72) Inventors:
• **Stremplewski, Patrycjusz**
**87-100 Torun (PL)**
• **Wojtkowski, Maciej**
**87-100 Torun (PL)**
• **Wnuk, Pawel**
**02-776 Warszawa (PL)**

(74) Representative: **JD&P Patent Attorneys Joanna Dargiewicz & Partners**
**Ul. Mysliborska 93A/50**
**03-185 Warszawa (PL)**

(56) References cited:
**US-A1- 2011 134 436   US-A1- 2014 022 554
US-A1- 2016 299 060**

• **Branislav Grajciar ET AL: "Parallel Fourier domain optical coherence tomography for in vivo measurement of the human eye"**, Optics express, 21 February 2005 (2005-02-21), pages 1131-48, XP055537813, United States DOI: 10.1364/OPEX.13.001131 Retrieved from the Internet: URL:https://www.osapublishing.org/DirectPD FAccess/2CE4F6F9-F011-64AA-0FDCE31EE672 5B7 7_82660/oe-13-4-1131.pdf?da=1&id=82660&seq =0&mobile=no [retrieved on 2018-12-21]
• **A E Desjardins ET AL: "Angle-resolved Optical Coherence Tomography with sequential angular selectivity for speckle reduction"**, Optics express, 14 May 2007 (2007-05-14), pages 6200-6209, XP055538538, United States DOI: 10.1364/OE.15.006200 Retrieved from the Internet: URL:https://www.osapublishing.org/DirectPD FAccess/8A92E223-F292-C559-31F61A4BF87A3 12 4_134561/oe-15-10-6200.pdf?da=1&id=134561& seq=0&mobile=no [retrieved on 2019-01-07]

- **Boris Povazay ET AL: "Full-field time-encoded frequency-domain optical coherence tomography", Optics express, 21 August 2006 (2006-08-21), page 7661, XP055537828, United States DOI: 10.1364/OE.14.007661 Retrieved from the Internet: URL:https://www.osapublishing.org/DirectPD FAccess/2D75D1DB-AB90-02DC-7CBDA4A0603 1422 9_97655/oe-14-17-7661.pdf?da=1&id=97655&se q=0&mobile=no [retrieved on 2018-12-21]**

# Description

## Field of the Invention

The invention relates to an apparatus for Optical Coherence Tomography imaging with parallel Fourier domain detection and a method of imaging by Optical Coherence Tomography with parallel Fourier domain detection, which can be used for in-vivo imaging, in particular for imaging of the retina and anterior chamber of the eye, but also for imaging of other organs, tissues and cells. The invention enables the reconstruction of the three-dimensional structure of objects without disturbances resulting from phase modulation of scattered light.

## Description of the Related Art

There is a known apparatus that is used to image cross-sections of transparent and partially transparent biological objects by means of Optical Coherence Tomography (OCT) with parallel detection, using a device that consists of a light source emitting light of multiple wavelengths which, after collimation, passes to an interferometer consisting of a beam splitter, a reference mirror, an optical system forming light on the object in the interferometer's sample arm and an optical system forming light on the reference mirror in the interferometer's reference arm. After reflection from the object and from the reference mirror, the light is registered by a multi-element photodetector which converts light into electrical signals that are further analyzed by a processing device. The method of imaging objects by means of this system is based on the interference of the beam of light reflected from the reference mirror with the beam of light scattered on the structural components of imaged object. The electrical signals obtained by the detector are processed by a computer. Parallel detection means that a multi-element photodetector is used and in a single exposure of the detector, linear or two-dimensional Optical Coherence Tomography data are recorded. Such detector can be a one or two-dimensional array of photosensitive components (for example a CMOS camera, CCD, linear CCD camera). When using a light source that emits many optical frequencies (wavelengths) the collection of this sort of data allows for the 3D reconstruction of the object (in the case of an array and a tunable laser source) or image cross-section (in the case of a line of detectors and a tunable laser source or an imaging spectrometer and a temporally incoherent light source). In this description, we will continue to use the universally recognized convention in which the Z axis coincides with the direction of light beam propagation (into the depth of the object) and the X and Y axes correspond to directions perpendicular to the direction of propagation of the beam illuminating the object (they symbolize directions on the surface of the object).

Due to the order of data collection and their type, there are various methods of performing Optical Coherence Tomography with parallel detection:

- Parallel Fourier domain detection in the plane (X,Z), where simultaneously a set of spectral interference fringes which correspond to one line in the plane (X,Y) is recorded. This method uses an temporally incoherent light source and an imaging spectrometer equipped with a two-dimensional photodetector array, as shown in publication B. Grajciar, et al. Parallel Fourier domain Optical Coherence Tomography for in vivo measurement of the human eye, Optics Express 13, 1131-1137 (2005), or a high-speed one-dimensional camera and a tunable laser (Swept Source) -this solution can be found in the publication Fechtig, D. J. et al. Line-field parallel swept source MHz OCT for structural and functional retinal imaging. Biomed. Opt. Express 6, 716 (2015);

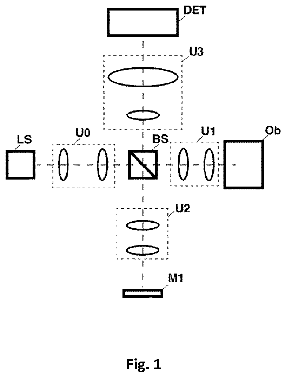

- Parallel Fourier domain detection in the plane (X,Y) in which each pixel of the 2D detector sequentially records spectral interference fringes created during the change of optical frequencies of a rapidly tunable laser (Fd-FF-OCT) - an example of its use is presented in the paper by Hillmann, D. et al. In vivo optical imaging of physiological responses to photostimulation in human photoreceptors. Proc. Natl. Acad. Sci.113, 13138-13143 (2016) and is shown in Figure 1;

- Parallel detection in the plane (X,Y) of signals in the time domain, in which an temporally incoherent radiation source and a two-dimensional photodetector array are used, as shown in the works of Boccara, C., Harms, F. & Latrive, A. Full-field OCT: a non-invasive tool for diagnosis and tissue selection. SPIE Newsroom 2-5 (2013). doi: 10.1117 / 2.1201306.004933 or Thouvenin, O., Apelian, C., Nahas, A., Fink, M. & Boccara, C. Full-Field Optical Coherence Tomography as a Diagnosis Tool: Recent Progress with Multimodal Imaging. Appl. Sci.7, 236 (2017). This method is traditionally called the Full field OCT technique and Td (Time domain - Td-FF-OCT) is added to mean that the signals are recorded as a function of the location of the reference mirror dependent on time. An example of the use of this technique can be found in the publication by Peng Xiao, Mathias Fink, and A. Claude Boccara, "Full-field spatially incoherent illumination interferometry: a spatial resolution almost insensitive to aberrations," Opt. Lett. 41, 3920-3923 (2016).

In all the Fourier methods described above (using a tunable laser or a spectrometer and temporally incoherent source) according to the embodiment shown in Figure 1, the reconstruction of the object in the Z direction is reduced to the transition from the frequency domain ($k_z=2\pi/\lambda$) to the spatial domain (Z) by means of Fourier transformation performed by the computer during or after data collection. In performing some operations on signals, for example such as numerical dispersion compensation, a complex representation of spectral interference fringes, in which there is information about the phase of the interference fringes, is used. A complex representation is obtained after adding the original signal of spectral interference fringes (as the real part) to its Hilbert transform (as the imaginary part). The complex signal representation can also be obtained by measuring at least three spectral interference fringe signals by phase shifting methods (Schwider, J. et al. Digital wave-front measuring interferometry: some systematic error sources. 22, 3421 (1983).) Complex representations of interferometric signals can be averaged in a complex manner by adding the real and imaginary parts separately and repeat presentation of the resultant signal as a complex number.

Usually in OCT systems, and in general in systems containing an interferometer, the shape of the interference fringes is the result of combining multiple optical fields that contain time-constant phase relations (stationarity of optical processes). Most of the known light detectors such as CCD cameras or CMOS cameras integrate signals during the exposure time of the camera, averaging many optical fields. This averaging process, taking place while the camera is recording and averaging the representation of complex signals, considers the phase relations of the interference fringes in the same manner, giving the same final result. Therefore, in the further part of the description, averaging of the spectra obtained directly from the measurement (by integrating signals on the camera during the recording process), shall also be referred to as complex averaging.

In contrast to complex averaging, when we make a transition from the frequency domain to the spatial domain and reconstruct the distribution of scattering centres in the object along the Z axis, as the square of the Fourier transform of the spectrum, and then sum up the data obtained in this way in several measurements, we obtain the average of the results of the image reconstruction. The result of this operation does not depend on the mutual phase of the signals subjected to averaging and leads to different results than complex averaging.

Known OCT systems with parallel detection and those operating on the basis of Fourier domain detection use spatially coherent radiation sources. Spatially incoherent sources are used in systems with parallel detection in the time domain, which are inherently less sensitive than systems with Fourier domain detection.

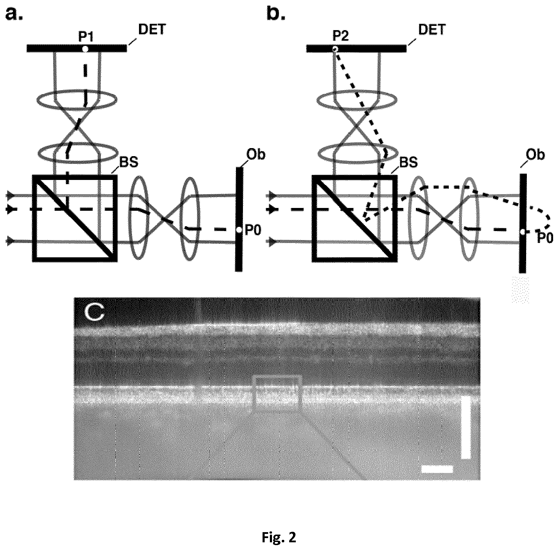

In OCT systems, the natural optical system of the eye is usually used as the component of the system forming light on the object, which is the retina of the eye. OCT techniques enable the visualization of retinal cross-sections in a non-invasive and noncontact way. The disadvantage of these methods and, in particular, methods with parallel Fourier domain detection is, however, the inability to visualize the deeper layers such as the choroid, which are located under the Retinal Pigment Epithelium (RPE) which strongly scatters light, preventing the tomographic reconstruction of the layers underneath. The problem of visualizing the deeper layers of the object, in the case of using spatially coherent sources in the system with parallel detection, is associated with the presence of a phenomenon called optical crosstalk, which limits the capabilities of imaging in objects that scatter light such as the choroid or skin. The creation of the crosstalk is illustrated in Fig. 2, where continuous and dashed lines are used to distinguish between the radiation from two areas of light source, which after passing through the beam splitter BS reach the object Ob, after reflection or scattering from the object they reach the optical system forming an image of the object on the camera array DET. Ideally, each of the distinguished rays contributes to the signal in only one allocated area of the detector, which does not coincide with an area assigned to any other rays (Fig. 2a). In practice, it is possible that part of the energy associated with one of the rays hits the area of the detector which should represent an area of the sample corresponding to the other ray, which is schematically indicated in Fig. 2b by means of a densely dashed line. Such a situation occurs in the presence of additional scatterings or phase disturbances in the optical system or the object itself. Usually the light source in the OCT system with parallel Fourier domain detection is characterized by high spatial coherence, which makes it impossible to distinguish whether the ray reaching the detector is assigned to the appropriate source area, thus creating optical inter-channel crosstalk. The phenomenon of optical crosstalk could be reduced by placing a diaphragm in the optical path, which would cut off the rays reaching the wrong place on the detector, however, it would limit the field of view recorded by the detector, e.g. the camera.

In OCT systems with parallel Fourier domain detection, no method has so far been developed to solve the above problem. At the same time, the crosstalk effect does not apply to Td-FF-OCT systems, where a light source emitting spatially incoherent radiation is used. However, the sensitivity of Td-FF-OCT systems is lower and they are much slower than OCT systems with parallel Fourier domain detection. Neither does this effect occur in OCT scanning systems, which inherently have a shield in the form of single-mode fiber or a single-element detector.

In techniques other than OCT, for example, in quantitative phase microscopy, in the system for the optical selection of the imaging plane that is applied to samples of considerable thickness, an interferometer is used and the sample is illuminated by a random light field. In practice, such illumination is achieved by means of a rotating diffuser placed in front of the interferometer and a laser with high temporal coherence (Choi, Y., Yang, TD, Lee, KJ & Choi, W. Full-field and single-shot quantitative phase microscopy using dynamic speckle illumination, Opt. Lett.36, 2465, 2011) or light sources with a wide emission spectrum (Choi Y. et al, Dynamic speckle illumination wide-field reflection phase microscopy. Opt.

Lett. 39, 6062-6065, 2014). However, an inconvenience in the technique of quantitative phase microscopy using dynamic illumination by a random light field is the small imaging depth range in the order of single micrometers. Another unfavorable feature of the technique of dynamic illumination by a random light field is the need to use a mechanical system regulating the length of the reference arm in order to select the imaged plane. In the present state of art, limitations in the speed of operation of devices introducing dynamic illumination with a random light field virtually eliminate the possibility of using quantitative phase microscopy systems for in-vivo imaging.

Moreover, the visualization of the deeper layers of the object, when using OCT with parallel Fourier domain detection, as with other OCT techniques, is affected by speckle noise that makes it difficult to distinguish details of images at the nominal resolution level of these systems, since the dimensions of the speckles are usually greater than or comparable to the resolution of OCT methods. This applies to both the image dimension in the plane perpendicular to the direction of light propagation (XY direction) as well as in the direction parallel to the direction of light propagation (Z direction). The mechanism of speckle formation is slightly different in these two dimensions and it is associated with the property of light coherence. The speckle image in the XY plane is dependent on the numerical aperture of the imaging system, whereas the speckle pattern in the Z direction depends on the central wavelength and the spectral width of the light source. In systems with parallel detection, there are two mechanisms leading to speckle noise. The first is the aforementioned optical crosstalk, the second mechanism is related to the limited spatial resolution of the imaging system. This second mechanism is also important in other types of OCT systems, which use the scanning of a beam focused on the object, e.g. in Spectral domain OCT or Swept Source OCT techniques. Within the distinguishable volume component (both optically in the X, Y plane, and temporally in the Z axis) there are many scattering centres. The total field scattered in such a volume component into a solid angle corresponding to the numerical aperture of the detector is the coherent superposition of the waves coming from the optically indistinguishable scattering centres within this volume. This coherent superposition means that a given volume component can be seen in the OCT reconstruction as a light or dark area.

In OCT with parallel Fourier domain detection no method has been developed so far to remove the aforementioned effect of speckle noise. However, methods for the reduction of speckle noise in OCT scanning systems are known, involving illuminating the sample from many angles and averaging the resulting two- or three-dimensional image reconstructions (image averaging). Changing the direction of illumination causes a change in the mutual phase of scattered radiation from individual scattering centres within the given volume element (the smallest distinguishable), averaging a correspondingly large number of such events leads to a reduction in the contrast of speckle noise. These methods have been used to eliminate speckle noise in imaging biological structures, such as, for example, Desjardins, E. et al. Angle-resolved Optical Coherence Tomography with sequential angular selectivity for speckle reduction. Opt. Express 15, 6200-6209 (2007) or in patent application WO2004/088361. The disadvantage of this method for OCT systems, which use the scanning of a beam focused on the object, is the limitation of the resolution of the imaging system due to the inability to use the full numerical aperture of the lens. This is due to the fact that in scanning systems, in order to introduce the possibility of changing the angle, there must be a room to change the location of the centre of the beam on the entrance pupil of the lens. This leads to a reduction in resolution in the plane (X, Y) in relation to the nominal value, achievable with a given lens.

The publication by Hillmann, D. et al. In vivo optical imaging of physiological responses to photostimulation in human photoreceptors. Proc. Natl. Acad. Sci. 113, 13138-13143 (2016), shows the use of Optical Coherence Tomography with parallel Fourier domain detection for the in-vivo imaging of human eye structures. Figure 2 in this publication (Fig. 2c) shows the image of retinal cross-sections strongly distorted by the effect of optical crosstalk and speckle noise. The crosstalk effect is visible in the form of a uniform background beneath the pigmented epithelial layer below, making it impossible to reconstruct details of the vascular layer. The effect of speckle, on the other hand, makes it impossible to distinguish individual cells, such as pigmented epithelial cells, 20 micrometers in size, despite a nominal resolution of 10 micrometers.

In the patent application US2011134436A1 methods and devices are disclosed for acquiring depth resolved aberration information using principles of low coherence interferometry and perform coherence gated wavefront sensing. The wavefront aberrations is collected using spectral domain low coherence interferometry or time domain low coherence interferometry principles. When using, chromatic aberrations can also be evaluated. Methods and devices are disclosed in using a wavefront corrector to compensate for the aberration information provided by CG-WFS in a combined imaging system, that can use one or more channels from the class of (i) optical coherence tomography, (ii) scanning laser ophthalmoscopy, (iii) microscopy, such as confocal or phase microscopy, (iv) multiphoton microscopy, such as harmonic generation and multiphoton absorption. In particular, a swept source (SS) is used that drives both an OCT channel and a coherence gated wavefront sensor, where: a) both channels operate according to SS-OCT principles; b) OCT channel integrates over at least one tuning scan of the swept source to provide a TD-OCT image of the object; c) CG-WFS integrates over at least one tuning scan of the swept source to provide an enface TD-OCT mapping of the wavefront. For some implementations, simultaneous and dynamic aberration measurements/correction with the imaging process is achieved. The methods and devices for depth resolved aberrations disclosed, will find applications in wavefront sensing and adaptive optics imaging systems that are more tolerant to stray reflections from optical interfaces, such as reflections from the microscope objectives and cover slip in microscopy and when imaging the eye, the reflection from the cornea.

In another patent application US20140022554A1 a method and apparatus are provided to generate tomography images that performs the method. The apparatus and method are configured to determine a basis pattern from modulated phases of incident rays from a spatial light modulator according to a pattern of arranged pixels. The apparatus and method are further configured to perform spatial shift modulation shifting an arrangement of the pixels vertically or horizontally with respect to the basis pattern to obtain shift patterns of the basis pattern. The apparatus and method are configured to generate tomography images for the basis pattern and the shift patterns using spectrum signals of rays obtained from the incident rays passing through the spatial light modulator and entering a subject. The apparatus and method are configured to select a pattern that generates a clearest tomography image of the subject based on the generated tomography images.

The American patent application US20160299060A1 discloses an immersion probe system for simultaneously performing first analysis of a first portion of light originating from liquids and/or particles in a fluid and second analysis of a second portion of the light originating from the liquids and/or particles. The system defines an optical axis and includes a first component including a first analyzer, a window, and a first optical path extending between the window and the first analyzer. The system also includes a second component including a second analyzer, the window, and a second optical path extending between the window and the second analyzer. The system further includes a spectral selector placed in the first optical path and in the second optical path to direct the first portion of the light, which originates from the liquids and/or particles and passes through the window, to the first analyzer, and to direct the second portion of said light to the second analyzer. The system includes an illumination path that delivers illumination light or lights based on a beam(s) that passes through the window at an oblique or normal angle to the optical axis. The first component and the second component share a common optical path at least between the window and the spectral selector.

In the publication "Parallel Fourier domain optical coherence tomography for in vivo measurement of the human eye" (Grajcar et al., 21 February 2005 / Vol. 13, No. 4 / OPTICS EXPRESS 1131) a parallel Frequency Domain Optical Coherence Tomography (FD-OCT) system and in vivo tomograms obtained with such system are presented. A full tomogram of 256(x) x 512(z) pixels covering a sample region of 8 mm x 3,8 mm is recorded in only 1 ms. Since the transverse as well as the depth information is obtained in parallel, the structure is free of any motion artifacts. In order to study cross talk issues for parallel illumination the transversal resolution for a thermal light source is compared to that with an SLD.

Summary of the invention

The invention relates to all OCT methods with parallel Fourier domain detection, which use a spatially coherent light source. In particular, it can be used in systems with parallel detection in the plane (X,Y) in which each pixel of a one- or two-dimensional detector, sequentially in time, records spectral interference fringes generated during the change of the optical frequencies of a rapidly tunable laser (Swept Source). It can also be used in systems with parallel detection in the plane (X,Z), where simultaneously a set of spectral interference fringes are recorded, which correspond to one line in the plane (X,Y) using an imaging spectrometer equipped with a two-dimensional photodetector array and a light source emitting spectral broadband radiation.

The aim of the invention is to improve the quality of the cross-sectional imaging using OCT with parallel Fourier domain detection, by a significant reduction of optical crosstalk. Optionally, the present invention also makes it possible to significantly reduce the contrast of speckle noise. An improvement in the quality of imaging by filtering out unwanted light which, as randomly scattered on the structure of the examined object, opens up the possibilities of imaging other, deeper biological structures than in classical OCT, in particular the possibility of imaging of the choroidal layer. The invention introduces a new imaging method including measurement and data analysis and a new apparatus as a measuring system using this method.

The invention achieves the above goal, i.e. the reduction of optical crosstalk through the unobvious use of tools typical of techniques for the optical selection of the imaging plane to OCT with parallel Fourier domain detection. The unobvious combination of these techniques also implies an unobvious modification of the design of the apparatus for OCT with parallel Fourier domain detection.

The subject of the invention is an apparatus for imaging objects by OCT with parallel Fourier domain detection, comprising a light source, a multi-element photodetector, an interferometer containing at least one beam splitter, a reference mirror, two optical systems and an optical system forming an image on a multi-element photodetector, characterized in that an instrument for spatially varying modulation of the phase of light and a device for independent control of the angular distribution of sample illumination are placed before beamsplitting means, configured to split the beam emitted by the light source into the reference beam and object beam

Preferably, the apparatus for spatially varying modulation of the phase of light comprises an active element selected from the group including a rotating or sliding diffuser, a mirror comprising a two-dimensional array of piezoelectric or liquid crystal elements or a vibrating light reflecting membrane, preferably in the form of a deformable mirror.

Preferably, the apparatus for changing the angles of illumination is: a DMD (Digital Micromirror Device) system, an acousto-optical deflector, a liquid crystal spatial light modulator, an electro-optical deflector, a MEMS (microelectromechanical system) or at least one resonance or galvanometric scanner equipped with at least one mirror mounted on at least one galvanometer engine. The device that enables changing the angles of illumination can also be any other system deflecting the light beam. The device may also be a liquid crystal spatial light modulator, which introduces into the cross-section of the beam a phase ramp in a different orientation and inclination resulting in changes in the angle of illumination of the sample. In this embodiment, the device introducing spatially varying modulation of the phase of light and the additional device to change the angles of illumination can be one device that performs both functionalities by simultaneously or sequentially applying the appropriate phase masks, for example phase ramps and random phase distributions. Also, changing the position of the light source in the plane Fourier-conjugated with the object plane can be used to change the angle of illumination of the sample.

Preferably, the optical system forming light on the object and the optical system forming light on the reference mirror comprise the same optical elements.

Preferably the device introducing spatially varying modulation is configured to modulate light phase, and the device for changing the illumination angles, configured to control the sample illumination angle, are physically separated and configured to operate independently.

Preferably, the detector is: a one-dimensional camera, a two-dimensional camera, a collection of photodiodes, or an imaging spectrometer that allows simultaneous recording of spectra for different points of the object.

In one preferred embodiment of the invention, where the multi-element photodetector is a line of photosensitive elements) or an imaging spectrometer, wherein an additional cylindrical optics is between the light source and the instrument, and a beam scanning device is in the interferometer object arm between the beam splitter and the optical system, for example a galvanometric scanner configured to deflect the beam in one direction. Then, between the beam splitter and the field forming system on the object, in the object arm of the interferometer, there is a beam scanning device, for example a galvanometric scanner configured to deflect the beam in one direction.

In another preferred embodiment, where the detector is a one-dimensional camera or a two-dimensional camera, the light source is a device allowing for a sweeping with optical frequencies or wavelengths with a spectral width greater than 0.5 nm. Then, when the detector is an imaging spectrometer the light source is a device allowing the generation of spatially coherent light with a spectrum with a spectral width greater than 0.5 nm.

The invention also relates to the method of OCT with parallel Fourier domain detection using a spectrally broadband light source, and multi-element photodetector used to create three-dimensional image reconstruction of the structure of the object, comprising steps of:

a) splitting the light by a beam splitter in a reference and object beams,
b) dynamic modulation of the phase of light spatially and temporally by using an instrument placed between the light source and the beam splitter

characterized in that dynamic spatial light phase modulation over the beam cross-section and independent control of the angular distribution of sample illumination are employed by devices for introducing spatially varying modulation and for changing the illumination angles before the beam emitted by the light source is split into the reference and object beams by the beamsplitter, and the interference images obtained by the photodetector are integrated in a complex manner.

Preferably, the phase values in the beam cross-section change randomly or according to a predetermined function.

Preferably, complex averaging is performed by integration on a multi-element detector at a given exposure time.

Preferably, complex averaging is performed by numerically adding the signals of complex representations of the interference images obtained from multiple measurements.

Preferably, the image of the beam cross-section in which the spatial phase modulation takes place, is formed in an object plane.

Preferably, the spatial frequency spectrum corresponding to the cross section of the beam in which the spatial phase modulation takes place is formed in an object plane

Preferably, successive interferometric images recorded sequentially over time by a multi-element detector correspond to different optical frequencies generated over time by a broadband light source.

Preferably, the interferometric images are recorded in the form of a set of spectra recorded by a multi-element light detector.

Preferably, the image reconstruction process is repeated for different illumination angles of the object and then the image reconstructions obtained for different illumination angles are averaged to obtain a final reconstruction of the three-dimensional image of the object.

Preferably, the light beam illuminating the object is formed into a line and spatial phase modulation is along the direction defined by the light beam formed into the line.

The subject of the invention is also the method for OCT with parallel Fourier domain detection as described above, using a device for imaging objects by the technique described above.

In the method according to the invention, OCT with parallel Fourier domain detection using a multi-element photodetector. The measurement method that is the subject of the present invention involves the recording of a plurality of interferometric images resulting from reflection or backscattering of the light from the object being examined and the reference mirror by means of a multi-element detector, where each of these images is measured when the phase of light entering the interferometer is modulated in the cross-section of the beam (spatially varying modulation of the phase of light). This modulation can be performed repeatedly during the exposure time of the camera, or sequentially for subsequent recorded interferometric images, which are later averaged in a complex manner during the numerical processing of the signals. In this way, for each narrow range of optical frequencies (a single frame recorded by the CMOS camera or a single line recorded by the imaging spectrometer), many interferometric signals resulting from the operation of the device introducing spatially varying modulation of the phase of light, are averaged in a complex manner. This complex signal averaging makes it possible to eliminate those components of the interferometric signal, which for variable phase of lights in the cross-section of the beam do not maintain a constant difference of optical paths when the object is stationary. This means that such signals correspond to accidentally or repeatedly scattered waves that do not carry information about the location of the object. The elimination of these components means a reduction in optical crosstalk and thus obtaining a more accurate image reconstruction. The set of interferometric images recorded for various optical frequencies is then saved in the PC computer memory or an external memory and undergoes further numerical processing analogous to the image reconstruction used in OCT with parallel Fourier domain detection.

Optionally, the entire procedure is repeated several times for different angles of illumination of the sample introduced by a device changing the illumination angles. Then, using this device, various interferometric signals are generated, for example by controlled deflection of the light beam in front of the interferometer, so that the change in the illumination angle is the same for the light illuminating the reference mirror and the measured object. The image reconstructions obtained in this manner are averaged giving the final form of image reconstruction free from the presence of undesirable effects i.e. optical crosstalk and speckle noise, which affect the imaging of three-dimensional objects, especially in-vivo imaging.

Various interferometric signals are generated by means of an element (device) introducing spatially varying modulation of the phase of light and can be carried out in two ways:

1. By imaging the active element of the device introducing spatial modulation of the phase of light on the surface or inside the test sample and on the surface of the reference mirror;

2. By creating a Fourier Spectrum of the light transmitted or reflected from the active element of the device introducing spatial modulation of the phase of light on the surface or inside the test sample and on the surface of the reference mirror.

The phase mask used to modulate the phase of light which is employed by the device introducing spatial modulation of the phase of light, may be a random phase distribution. In this case, in order to obtain a better performance of the system, the size of the smallest fixed phase element in a given random mask should match the numerical aperture of the system so that the light scattered due to such phase modulation is not vignetted by the elements of the optical system. An effect analogous to the elimination of optical crosstalk can be obtained by introducing deterministic phase masks used for phase modulation, given by a device introducing phase modulation, such as, for example, a time-variable array of Hadamard phase masks or Zernike polynomials. It is also possible to functionally combine the operation of the device introducing spatial light modulation and the device changing the angle of illumination of the sample by using liquid crystal spatial light modulators that simultaneously or sequentially modulate the phase, using dedicated phase masks imposed on phase ramps with variable phase slope and orientation in the plane perpendicular to the direction of light propagation, which allow for changing of the angle of illumination of light illuminating the sample and the reference mirror with simultaneous spatially varying modulation of the phase of light.

Other methods for introducing spatially varying modulation of the phase of light include the use of any light-reflective membrane that is vibrated. Such a device has enormous practical importance due to its ability to operate at high modulation rates. Another, less practical version of the above idea is the use of a rotating diffuser as the instrument introducing spatially varying modulation of the phase of light. This is a solution known from the literature and widely available, however its action may be strongly limited due to the considerable thickness of the scattering medium and the finite rate of phase modulation. In the less demanding applications of the invention, it is also possible to use a reflecting array of piezoelectric elements that spatially modulate the phase of light or a liquid crystal phase modulator, both in reflection and transmission mode.

The interferometer system can be designed in many ways, including typical interferometers such as the Michelson, Twyman-Green, Linnik or Mach-Zender interferometers. In another, alternative configuration, the transmission object may be in one of the arms of the Mach-Zender interferometer. In addition, a fiber-optic interferometric device is possible - but the fiber optic used here must be multimode or it must be a bundle of single-mode optical fibers.

Another possible embodiment of the apparatus is the use of a light source emitting short femtosecond pulses or Amplified Spontaneous Emission light (ASE) with a spectral width greater than 0.5 nm. In this case, the multi-element light detector may be an imaging spectrometer, i.e. a device that measures light spectra for successive points of a one-dimensional image of the sample. In this case, the system forming the light field on the object together with the system forming the light field on the reference mirror form a beam of light in a line that illuminates the object and the reference mirror. The linear images of the surface of the reference mirror and the object are mapped in one dimension in the system of the imaging spectrometer, while the second dimension enables the separation of the components of the optical wavelengths. In practice, this is accomplished by using at least one cylindrical lens, a slit or a suitable diffractive element.

In a different embodiment of the system, it is possible to map the spectrum of spatial frequencies (planes in Fourier space) corresponding to the image of the surface of the deformable mirror or any spatial light modulator respectively on the plane of the reference mirror and the object by means of twin systems for forming light on the object and the reference mirror that can be four-lens systems.

The image forming systems inside the interferometer can consist of identical optical elements creating the Linnik interferometer system. In the case of retinal imaging of the human eye, the natural optical system of the eye becomes a component of the optical system forming light on the object whilst the optical system forming light on the reference mirror has optical elements mimicking the optical parameters of the eye's optical system in such a way that the optical system in the reference arm and the imaging system together with the human eye are equivalent.

### A brief description of the drawings

The invention will now be further illustrated in the preferred embodiments, with reference to the accompanying drawings, in which:

Fig. 1 shows a simplified scheme of devices for Optical Coherence Tomography with parallel Fourier domain detection, which are known in the current state of the art exemplified by the work of Hillmann, D. et al. In vivo optical imaging of physiological responses to photostimulation in human photoreceptors. Proc. Natl. Acad. Sci.113, 13138-13143 (2016);

Fig. 2 shows the occurrence of optical crosstalk in known OCT apparatus with parallel Fourier domain detection: a. A case of perfect image mapping, b. A case of optical crosstalk, c. An example cross-section image with optical crosstalk from the paper by Hillmann, D. et al. In vivo optical imaging of physiological responses to photostimulation in human photoreceptors. Proc. Natl. Acad. Sci.113, 13138-13143 (2016);

Fig. 3 shows an example of an embodiment of the apparatus that is not part of the claimed invention;

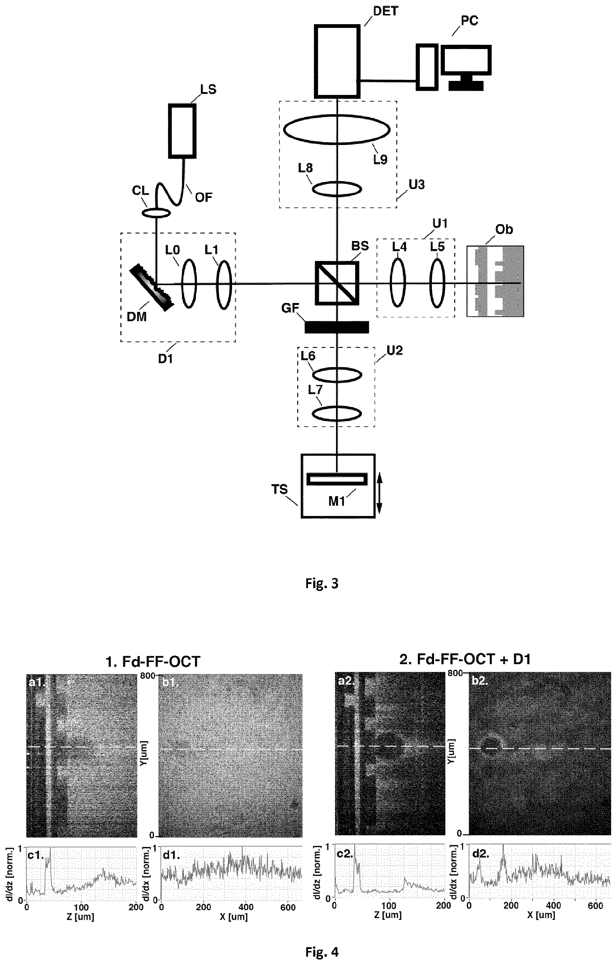

Fig. 4 shows results illustrating the improvement in quality of OCT imaging due to the use of the apparatus and method in relation to the state of the art where: 1. Shows images of cross-sections obtained by the OCT method with parallel Fourier domain detection in the mode known from the state of the art (Fd-FF-OCT); 2. Shows devices using a device D1 for phase modulation allowing for the elimination of crosstalk (Fd-FF-OCT + D1); The object Ob being imaged is a tissue phantom with a layered structure. The images on the panels (a) show transverse cross-sections parallel to the direction of light propagation (in the Y-Z plane), images on the panels (b) show cross-sections in the plane transverse to the direction of light propagation (XY) at a depth of 100 micrometers below the surface of the test sample. Images (c) and (d) show the derivatives of the spatial distribution of the intensity of the backward reflected signal from the sample along the lines marked on pictures a and b;

Fig. 5 shows an embodiment of the apparatus , which is not part of the claimed invention, intended for imaging the retina of the eye;

Fig. 6 shows an embodiment of the apparatus, which is not part of the claimed invention, where the illumination of the object is formed into a line;

Fig. 7 illustrates an embodiment of the apparatus, which is not part of the claimed invention, when using a system of an imaging spectrometer and a broadband light source;

Fig. 8 shows an embodiment of the apparatus according to the invention when the system is equipped with a device for introducing variable angles of illumination of the object,

Fig. 9 illustrates an embodiment of the apparatus according to the invention for imaging the retina when the system is equipped with a device for introducing variable angles of illumination of the object;

Fig. 10 shows an embodiment of the apparatus according to the invention where the system is additionally equipped with an instrument introducing variable angles of illumination of the object and the illumination of the object is formed into a line;

Fig.11 depicts an embodiment of the apparatus according to the invention where an imaging spectrometer and a broadband light source are used as well as an additional device introducing variable angles of illumination of the object;

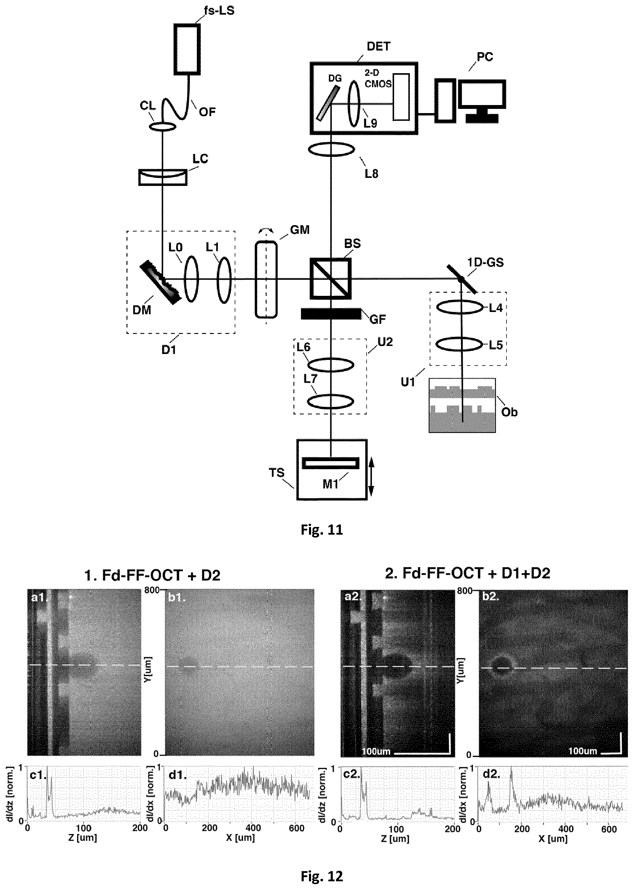

Fig. 12 shows results illustrating a further improvement in the quality of OCT imaging by using the apparatus and method that are the subject of the invention, in combination with a variable angle of illumination of the object: 1. Cross-sectional images obtained by means of OCT with parallel Fourier domain detection using the instrument D2 to change the angle of illumination of the sample (FD-OCT-FF+D2); 2. Cross-sectional images obtained with the apparatus according to the invention using the device D1 and the device D2 (Fd-FF-OCT + D1 + D2). The object Ob being imaged is a tissue phantom with a layered structure. The images on the panels (a) show transverse cross-sections parallel to the direction of light propagation (in the Y-Z plane), the images on the panels (b) show cross-sections in the plane transverse to the direction of light propagation (XY) at a depth of 100 micrometers below the surface of the test sample. Images (c) and (d) show the derivatives of the spatial distribution of the intensity of the backward reflected signal from the sample along the lines marked on the images a and b.

Examples 1 to 4 and figures 1 to 7 are not encompassed by the wording of the claims but are considered as useful for understanding the invention.

Key to the symbols used in the description: OB - object; DET - detector (photodetector); D1 - device (component) introducing phase modulation (for phase modulation), the so-called spatial light modulator; D2 - device (component) for changing the illumination angles; M1 - reference mirror; LS - light source; BS - beam splitter; L0-L12 achromatic biconvex lenses; OF - optical fiber, U0-U3 optical sets forming a beam; PC - computer; GM - galvanometric scanner, DM - deformable mirror; CL - collimator; CMOS - fast camera; L-CMOS - fast camera with a line of detectors (when the DET detector is in the form of a line of light-sensitive elements); P0-2 - light source image mapping points; 1D-GS - galvanometric scanner deflecting the beam in one direction; LC - cylindrical lens, IS - imaging spectrometer, DG - diffraction grating, 2-D CMOS - camera with two-dimensional array of detectors, fs-LS - broadband light source, GF - neutral gradient filter.

Detailed description of the embodiments of the invention

Example 1

The optical system depicted in Figure 3 was employed to carry out the measurement method, which is not part of the claimed invention, consisting of the recording of a plurality of interferometric images measured during the modulation of the spatial phase of light entering the interferometer system. A key element of the presented method is the Dyoptyka DM phase-randomizing deformable mirror with a unique design. This mirror is a thin membrane that vibrates at a frequency of above 200 kHz. These vibrations are induced in conditions that are different to those for obtaining transverse resonance modes on the surface of the membrane. As shown in Fig. 3, the phase-randomizing deformable mirror DM, which together with lenses L0 and L1 with 50mm focal length performs the function of the device D1, is placed in the path of the light beam, formed by the collimator CL, emitted from the light source LS through a single-mode optical fiber OF. In this system the light source LS is the Superlum Swept Source Broadsweeper rapidly tunable laser, which allows for rapid sweep of optical frequencies, in a band of about 70 nm with a central wavelength of 840 nm in the spatially generated coherent beam of light. In order to obtain interferometric images, the spatially modulated beam is split by the beam splitter BS (Thorlabs BS014) and half of the energy is directed to the reference arm of the interferometer and the other half to the object arm. Images of the surface of the deformable mirror DM are formed respectively in the plane of the reference mirror M1 and in the plane of object Ob using two identical optical systems U1 and U2 consisting of pairs of achromatic lenses L4/L5 and L6/L7 also arranged in a 4f system with focal lengths respectively: L4 = 50mm, L5 = 30mm, L6 = 50mm, L7 = 30mm. After attenuation by the neutral gradient filter GF and reflection from the reference mirror M1 installed on the translational table and after scattering on the object Ob, images of the mirror M1 and the object Ob are formed on the multi-element light detector DET, which is a Photron FastCam CMOS fast camera. The images are formed by the optical system U3 consisting of two achromatic lenses L8, L9 with lengths: L8 = 50mm and L9 = 250mm. As a result, a set of interferometric signals is created on the CMOS camera which is specific to each of the optical frequencies of the waves generated by the tunable laser LS and for each phase modulation by the deformable mirror DM of the device D1. This is possible due to the fact that the phase is spatially modulated by the membrane fluctuations in a time shorter than the exposure time of the CMOS camera - in this example the phase change takes place in less than 5 microseconds. Therefore, the interference image recorded by the CMOS camera for one optical frequency of the wave generated by the swept source LS is the result of the averaging of many interferometric signals. Since this averaging takes place during the opening of the CMOS camera shutter (complex averaging) the phase relations in the beams from the reference arm and the object arm of the interferometer are retained only for those waves that do not undergo strong distortions of the wavefront in one of the interferometer arms. Distorted waves, which are responsible for the crosstalk effect, interfere with variable phases, meaning that the visibility of the interferometric fringes from these waves is less than imaging noise. On the same principle, new, crosstalk-free interference images are recorded by the CMOS camera for successive optical frequencies generated by the light source LS. Recording of interference images is repeated many times for different optical frequencies until a set of signals enabling the reconstruction of the three-dimensional structure of the object Ob in a manner typical of the OCT technique with parallel Fourier domain detection is obtained.

The measuring process involves the following example sequence of activities:

1. The rapidly tuned laser LS and the high-speed CMOS camera are simultaneously triggered;

2. The laser LS begins to change the wavelength of emitted radiation at a rate of 4500 nm/s, starting at 878 nm, ending at 801.2 nm, the CMOS camera begins to acquire 1024 images with a resolution of 512 x 512 pixels at a rate of 60,000 frames/s, which is collected in its internal memory - the recording of a single set of data for OCT reconstruction commences;

3. The collimated beam from the tuned laser LS illuminates the surface of the phase-randomizing deformable mirror DM;

4. The phase-randomizing deformable mirror D1 operates in continuous mode with a frequency of hundreds of kilohertz - for each irradiation of the CMOS camera lasting 16 microseconds there are several periods of wave front modulation;

5. The image of the surface of the deformable mirror DM is formed by the optical system U1 on the object Ob and by the optical system U2 on the surface of the mirror M1 in the reference arm of the interferometer;

6. The optical waves scattered on the object Ob and reflected from the reference mirror M1 are transmitted by the optical system to the surface of the CMOS camera array, where they interfere creating interferometric signals;

7. During one exposure, the CMOS camera records the interferometric signal corresponding to a narrow spectrum of the laser LS: $\Delta\lambda = 4500nm/s*16*10^{-6}$ s= 0.072 nm;

8. Once the 1024 frames have been recorded, the CMOS camera sends data to the personal computer PC where they are saved to the hard disk.

The signal processing required to reconstruct the structure of the tested object is identical to the method of analysis of data obtained by OCT with parallel Fourier domain detection, which is known from the literature.

Figure 4 shows the result of the above described measuring process, which is not part of the claimed invention, along with the result obtained by means of the device according to the scheme known from the state of the art (Fig 1). The measurement was carried out using a phantom consisting of two scattering layers immersed in a transparent material as the object Ob. One scattering layer with a thickness of about 15 microns is above the second layer, the thickness of which is a few millimeters. There are two "ICHF" logotypes on the surface of the scattering layers that are offset from each other. These logotypes are also made of the same scattering material. In the pictures of cross-sections Fig.4.a1 and Fig.4.a2 the logotype fragments have the shape of rectangular blocks growing out of the scattering layers. In addition, in the thicker layer of the diffuser there is a spherical cavity with a diameter of about 50 micrometers filled with a transparent material surrounded by the diffuser. Pictures (Fig.4a1 and Fig. 4.a2) show transverse cross-sections parallel to the direction of light propagation (in the Y-Z plane), pictures (Fig. 4.b1, 4.b2) show cross-sections in the plane transverse to the direction of light propagation (XY) at a depth of 100 micrometers below the surface of the test sample. For each of these images, a quantitative analysis of the local contrast was performed by counting the derivative of the spatial distribution of the intensity of the backscattered signal on the sample along the lines marked on pictures a and b (Fig 4.c1, 4.c2). This analysis makes it possible to visualize the edges of the objects - if the contrast is high and the edges are clearly visible, then the derivative signal should show a peak at the site of the expected edge. In the case of the analyzed structure, it was assumed that the edges of the cavity located in the scattering layer should be visible on the derivative signals. When using the existing unmodified OCT technique with parallel Fourier domain detection, the edges of the cavity are not visible either in the YZ projection or in the XY projection (Fig. 4.c1, Fig. 4.d1). After applying spatially varying modulation of the phase of light, the edges of the cavity are clearly visible in both projections (Fig. 4.c2, Fig. 4.d2). The use of derivative analysis of the spatial distribution of the intensity of the backward reflected signal from the sample allows a quantitative assessment of the improvement in the contrast of image reconstruction. In this case, a three-fold improvement in contrast was achieved, which made it possible to recognize the structure of the cavity surrounded by the light scattering material. The use of the spatial modulator D1 enabled the elimination of the crosstalk effects manifested by a reduced contrast of the details of the components of the object located in the highly scattering layer (the spherical cavity).

Example 2

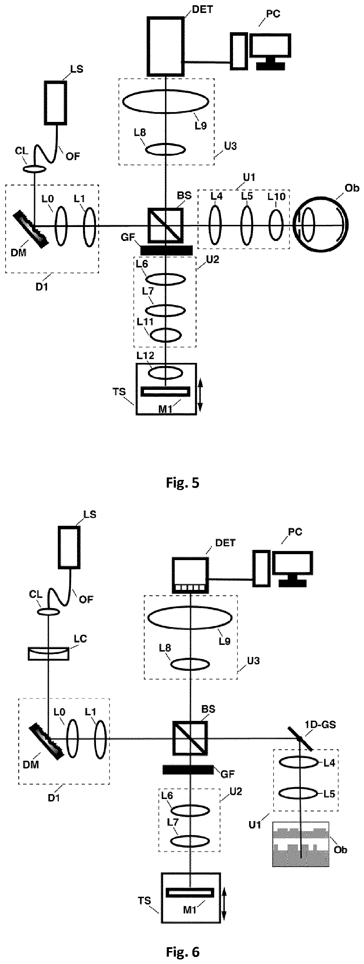

The apparatus described in Example 1 is modified for human retinal imaging, as illustrated in Fig. 5. The optical systems U1 and U2 are adapted to the natural optics of the eye due to the fact that U1 has three achromatic lenses L4, L5 and L10 arranged in such a way that the plane of the entrance pupil of the imaging system coincides with the plane of the pupil of the human eye. U2 has an additional lens L11 and in front of the reference mirror M1 there is another lens L12 with a focal length close to the focal length of the natural optical system of the eye. The apparatus was used for retinal imaging and this was also achieved by abandonment of the L5, L10 and L7 and L11 lenses at the expense of less flexibility in the selection of enlargement on the retina.

Example 3

The apparatus described in Example 1 was used and the imaging method was OCT with parallel Fourier domain detection using this apparatus where the sample illumination is in the shape of a line and the light detector system DET is a one-dimensional L-CMOS camera, which is illustrated in Fig. 6. In front of the deformable mirror DM, there is a cylindrical lens LC that forms a beam in the shape of a line on its surface. In this system, three-dimensional reconstruction is achieved by placing an additional galvanometric scanner 1D-GS in front of the optical system U1, which allows for sweeping the line illuminating the object in a direction perpendicular to the direction determined by this line.

Example 4

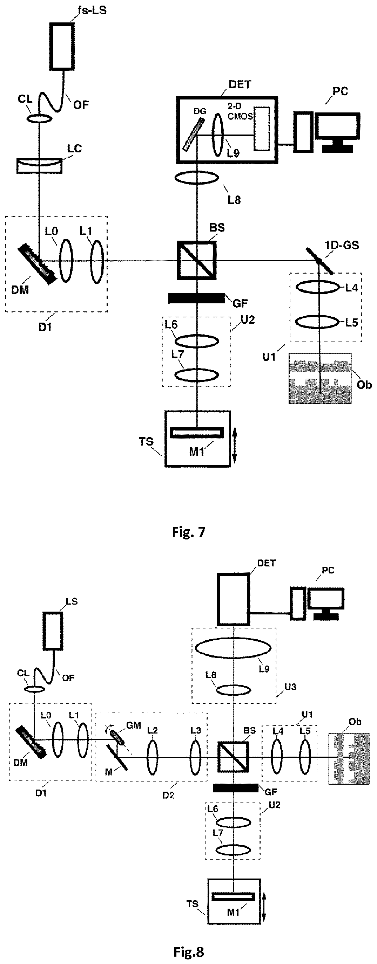

Fig. 7 shows a further modification of the apparatus in Example 1, in which instead of using a rapid tunable swept source laser, a spectral broadband light source LS emitting radiation containing a plurality of component optical frequencies is used. In this embodiment, this is a Femtolasers Fusion femtosecond laser fs-LS generating 6fs pulses with an output power of 300mW. In this case, the modification of the optical system involves changing the detector from a line of photosensitive elements to an imaging spectrometer IS, including a camera with a 2-D CMOS array and an efficient Wasatch Photonics holographic diffraction grating DG (800 lines/mm). In this embodiment, similarly to the one shown in Fig. 6, the light illuminating the sample is formed into a line by means of a cylindrical lens LC, and the three-dimensional reconstruction is achieved by placing an additional galvanometer scanner 1D-GS in front of the system U1, which allows for sweeping the object Ob with an illuminating line in a direction perpendicular to the direction designated by this line.

Example 5

The process of creating image reconstructions from Example 1 was repeated for different angles of illumination of the object Ob. The modified apparatus shown in Example 1 was used for this, illustrated in Fig. 8, in which there is additionally a device D2 for changing the illumination angles of the sample beam, which is a pair of Thorlabs galvanometric scanners GM additionally equipped with a mirror M. In the apparatus according to Fig. 8 a fast-response deformable mirror DM was used together with lenses L0 and L1 as the device D1 introducing phase modulation and a galvanometer scanner GM or 1D-GS together with lenses L2 and L3 as the device D2 to change the angle of incidence of the light illuminating the sample. In this embodiment, a Broadsweeper fast tunable laser TL was used as the light source. The reference mirror M1 was installed on a translational stage that allows for precise positioning of the device. The detector in this system is a fast CMOS camera. The device D2 is placed after the laser LS and the device D1 comprising phase-randomizing deformable mirror DM. Light from the laser LS illuminates the deformable mirror DM, introducing spatially varying modulation of the phase of light. Further on, the beam falls on the mirror of the galvanometer scanner GM or 1D-GS.

The plane of the galvanometer scanner mirror GM or 1D-GS is optically conjugate to the plane corresponding to the surface of the deformable mirror DM. To control the angle of illumination of both: the object Ob and the reference mirror M1 without vignetting on the beam splitter BS, two lenses L2, L3 operating in the 4f system were used with focal lengths respectively: L2 = 50mm, L3 = 50mm. These lenses image the pivot point of the scanner GM in the plane located in the middle of the beam splitter BS. The final image of the object and the reference mirror is formed by the optical system U3 comprising the above two lenses L8 and L9 arranged in the 4f configuration, on the detector DET. As in Example 1, as a result of this imaging, a set of interferometric signals is created on the CMOS camera which is specific to each of the wavelengths generated by the tunable light source LS for each phase modulation introduced by the deformable mirror DM. This time, however, the entire measurement containing a set of interferometric images obtained for various optical frequencies is repeated for several positions of the mirror of the galvanometric scanners GM in the instrument D2. Similarly to example 1 analysis of the effectiveness of reducing the contrast of speckle noise was carried out using a tissue phantom comprising two layers of highly scattering light material. Figure 12.1 shows the result of the imaging method when only the instrument D2 is used to change the angles of illumination of the sample Ob. These images were reconstructed by averaging 10 three-dimensional reconstructions recorded for 10 different positions of the galvanometer scanner GM or 1D-GS. The contrast of the image in the highly scattering layer improved slightly, but the presence of crosstalk makes it impossible to distinguish the edge of the cavity in signals derived from the spatial distribution of the back- reflected intensity (object Ob) measured along one line of the image (Figs. 12.c1,12.d1) . Fig. 12.2 shows the results of the operation of both devices, for spatially varying modulation of phase D1 and for changing the angle of illumination, D2. In this case, not only the improvement of the imaging contrast due to the reduction of crosstalk is visible but additionally there is a reduction in the contrast of the speckle noise manifesting in reduced graininess and better visibility of the details of Figs. 12.a2, 12.b2. In order to quantitatively analyze the reduction of contrast of the speckle noise, the ratio of the contrast-to-noise ratio was calculated, defined as:

$$CNR = \frac{\overline{I_S} - \overline{I_N}}{\sqrt{\sigma_S^2 + \sigma_N^2}}$$

where: $I_S$, $I_N$ are average signal and noise intensities, $\sigma_S$ and $\sigma_N$ are standard deviations of signal and noise. After manual selection of the signal area and noise, we obtain CNR = 1.37 for the data shown in Fig. 12.1 corresponding to the use of only the phase change device, D1 and CNR = 2.1 for the data shown in Fig. 12.2 corresponding to the use of the devices D1 and D2. For both reconstructions, $I_S$ and $I_N$ are similar ($I_S$ -$I_N$ for the data shown in Fig. 12.1 = 2460, $I_S$ -IN for the data shown in Fig. 12.2 = 2195). This means that a significant increase of CNR is associated to efficient suppression of speckle contrast.

Example 6

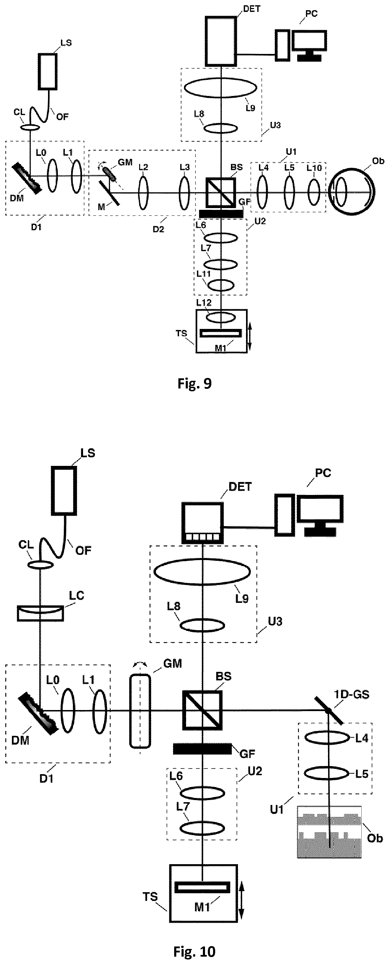

The apparatus and method for imaging according to the apparatus shown in Examples 1, 2 and 5 has been modified for the purposes of human retinal imaging in accordance with Fig. 9.

Example 7

Another example of the apparatus and the method of OCT with parallel Fourier domain detection using the apparatus according to the invention is shown in Fig. 10, which is a modification of the apparatus described in Example 5, the modification involves the illumination of the object Ob (the sample) in the shape of a line and the light detector system DET is a one-dimensional camera. In addition, in contrast to Example 5, the galvanometer scanner GM operates in one dimension with the scan direction perpendicular to the direction determined by the scanner 1d-GS.

Example 8

A further embodiment of the apparatus and a method of imaging with OCT with parallel Fourier domain detection according to the invention is illustrated in Fig. 11. The apparatus is a modified system (apparatus) from Example 4 (Fig. 7). The apparatus works with the use of a spectral broadband LS light source emitting radiation containing many optical component frequencies. As in Example 7, a galvanometer scanner GM used as D2 operates in one dimension with the scanning direction perpendicular to the direction determined by the scanner 1d-GS.

**Claims**

1. An apparatus for imaging objects by OCT with parallel Fourier domain detection, comprising a light source (LS), a multi-element photo-detector (DET), an interferometer containing at least one beam splitter (BS), a reference mirror (M1), two optical systems U1, U2 and an optical system (U3) forming an image on the multi-element photodetector (DET), **characterized in that** an instrument (D1) for spatially varying modulation of the phase of light and a device (D2) for independent control of the angular distribution of sample illumination are placed before the at least one beam splitter (BS), configured to split the beam emitted by the light source into a reference beam and an object beam.

2. An apparatus according to Claim 1, **characterized in that** the device (D1) introducing spatially varying modulation of the phase of light comprises an active element selected from the group containing a rotating or sliding diffuser, a mirror comprising a two-dimensional array of piezoelectric or liquid crystal elements or a vibrating membrane allowing to reflect light, preferably in the form of a deformable mirror (DM).

3. An apparatus according to Claim 1, **characterized in that** the device (D2) for changing the illumination angles is a digital micromirror device DMD, an acoustooptic deflector, an electro-optic deflector, a liquid crystal spatial light modulator, a Micro Electro Mechanical System (MEMS) mirror or at least one resonant or galvanometric scanner (GM) equipped with at least one mirror mounted on at least one galvanometer engine.

4. An apparatus according to any of the preceding Claims from 1 to 2, **characterized in that** the instrument (D1), configured to modulate light phase, and the device (D2), configured to control the sample illumination angle, are physically separated and configured to operate independently.

5. An apparatus according to any of the preceding Claims from 1 to 4, **characterized in that** the optical system U1 forming light on the object (Ob) and the optical system U2 forming light on the reference mirror (M1) comprise similar optical elements.

6. An apparatus according to any one of the preceding Claims from 1 to 5, **characterized in that** the multi-element photodetector (DET) is a one-dimensional camera (L-CMOS), a two-dimensional camera (CMOS), a photodiode array or imaging spectrometer (IS) enabling the simultaneous recording of spectra by mapping them for different points of the object (Ob).

7. An apparatus according to any one of the preceding Claims from 1 to 6, **characterized in that** the multi-element photodetector (DET) is a line of photosensitive elements (L-CMOS) or an imaging spectrometer (IS), wherein an additional cylindrical optics is between the light source (LS) and the instrument (D1), and a beam scanning device is in the interferometer object arm between the at least one beam splitter (BS) and the optical system U1, the beam scanning device being for example a galvanometric scanner configured to deflect the beam in one direction (1D-GS).

8. An apparatus according to any one of the preceding Claims from 1 to 7, **characterized in that** the multi-element photodetector (DET) is a one-dimensional camera (L-CMOS) or a two-dimensional camera (CMOS), the light source (LS) is a device allowing for a sweep of optical frequencies or wavelengths with a sweep span covering a spectral bandwidth greater than 0.5 nm.

9. An apparatus according to Claim 7, **characterized in that** the multi-element photodetector (DET) is an imaging spectrometer (IS), the light source (LS) is a device allowing for the generation of spatially coherent light (fs-LS) with a spectrum spanned over a bandwidth greater than 0.5 nm.

10. A method of imaging using Optical Coherence Tomography with parallel Fourier domain detection using a spectrally broadband light source (LS), and multi-element photodetector (DET) used to create three-dimensional image reconstruction of the structure of the object (Ob), comprising steps of:

    a) splitting the light by a beam splitter (BS) in a reference and object beams,
    b) dynamic modulation of the phase of light spatially and temporally by using an instrument (D1) and a device (D2) placed between the light source and the beam splitter

    **characterized in that** the instrument (D1) provides dynamic spatial light phase modulation over the beam cross-section and the device (D2) provides independent control of the angular distribution of sample illumination before the beam emitted by the light source (LS) is split into the reference and object beams by the beamsplitter (BS), and the interference images obtained by the multi-element photodetector (DET) are integrated in a complex manner.

11. The method according to Claim 10, **characterized in that** the phase values in the beam cross-section change randomly or according to a predetermined function.

12. The method according to Claim 10 or 11, **characterized in that** complex averaging is performed by integration on a multi-element photodetector (DET) at a given exposure time.

13. A method according to any one of the preceding Claims from 10 to 12, **characterized in that** complex averaging is performed by numerical summation of complex representations of interference images obtained from multiple measurements.

14. The method according to any one of the preceding Claims from 10 to 13, **characterized in that** the image of the beam cross-section, in which the spatial phase modulation takes place, is formed in an object plane.

15. The method according to any one of the preceding Claims from 10 to 14, **characterized in that** the spatial frequency spectrum corresponding to the cross-section of the beam in which spatial phase modulation occurs is formed in an object plane.

16. The method according to any one of the preceding Claims from 10 to 15, **characterized in that** the successive interferometric images recorded sequentially over time by the multi-element photodetector (DET) correspond to the different optical frequencies generated over time by the broadband light source (LS).

17. The method according to any one of the preceding Claims from 10 to 16, **characterized in that** the interferometric images are recorded in the form of a set of spectra recorded by a multi-element photodetector (DET).

18. The method according to any one of the preceding Claims from 10 to 17, **characterized in that** the process of image reconstruction is repeated for different illumination angles of the object (Ob) and then the image reconstructions obtained for different illumination angles are averaged to obtain a final image reconstruction of the three-dimensional structure of the object (Ob).

19. The method according to any one of the preceding Claims 10 to 18, **characterized in that** the light beam illuminating the object (Ob) is formed into a line and the spatial phase modulation is carried out along the direction defined by the light beam formed in a line.

20. The imaging method according to any one of the preceding Claims 10 to 19 carried out in the apparatus according to any one of the preceding Claims 1 to 9.

**Patentansprüche**

1. Vorrichtung zum Abbilden von Objekten mit Hilfe von OCT mit paralleler Fourierdomänendetektion, umfassend eine Lichtquelle (LS), einen mehrteiligen Fotodetektor (DET), ein Interferometer, das mindestens einen Strahlteiler (BS), einen Referenzspiegel (M1), zwei optische Systeme U1, U2 und ein auf dem Multielement-Fotodetektor (DET) ein Bild erzeugendes optisches System (U3), enthält, **dadurch gekennzeichnet, dass** eine Vorrichtung (D1) zur räumlich variierenden Modulation der Lichtphase im Strahlquerschnitt und eine Vorrichtung (D2) zur unabhängigen Steuerung der Winkelverteilung der Probenbeleuchtung vor dem mindestens einen Strahlteiler (BS) angeordnet sind, der so konfiguriert ist, um den von der Lichtquelle emittierten Strahl in einen Referenzstrahl und einen Objektstrahl aufzuteilen.

2. Vorrichtung nach Anspruch 1, **dadurch gekennzeichnet, dass** die Vorrichtung (D1), die eine räumlich variierende Modulation der Lichtphase einführt, ein aktives Element umfasst, das aus der Gruppe ausgewählt ist, die einen rotierenden oder verschiebbaren Diffusor, einen eine zweidimensionale Anordnung von piezoelektrischen oder Flüssigkristallelementen enthaltenden Spiegel, oder eine das Licht reflektierende vibrierende Membran, vorzugsweise in Form eines deformierbaren Spiegels (DM), umfasst.

3. Vorrichtung nach Anspruch 1, **dadurch gekennzeichnet, dass** die Vorrichtung (D2) zum Ändern der Beleuchtungswinkel eine digitale Mikrospiegeleinrichtung DMD, ein akustooptischer Deflektor, ein elektrooptischer Deflektor, ein Flüssigkristall-Raumlichtmodulator, ein elektromechanisches Spiegel-Mikrosystem (MEMS) oder mindestens ein resonanter oder galvanometrischer Scanner (GM), der mit mindestens einem, an mindestens einem Galvanometermotor montierten Spiegel ausgestattet ist, ist.

4. Vorrichtung nach einem der vorangehenden Ansprüche 1 bis 2, **dadurch gekennzeichnet, dass** die zur Modulation der Lichtphase konfigurierte Vorrichtung (D1) und die zur Steuerung des Probenbeleuchtungswinkels konfigurierte Vorrichtung (D2) physikalisch getrennt und separat konfiguriert sind, um unabhängig voneinander zu arbeiten.

5. Vorrichtung nach einem der vorhergehenden Ansprüche 1 bis 4, **dadurch gekennzeichnet, dass** das das Licht auf dem Objekt (Ob) bildende optische System U1 und das das Licht auf dem Referenzspiegel (M1) bildende optische System U2 ähnliche optische Elemente umfassen.

6. Vorrichtung nach einem der vorangehenden Ansprüche 1 bis 5, **dadurch gekennzeichnet, dass** der mehrteilige Fotodetektor (DET) eine eindimensionale Kamera (LCMOS), eine zweidimensionale Kamera (CMOS), eine Fotodiodenanordnung oder ein bildgebendes Spektrometer (IS), das die gleichzeitige Aufnahme von Spektren ermöglicht, indem sie für verschiedene Punkte des Objekts (Ob) abgebildet werden, ist.

7. Vorrichtung nach einem der vorangehenden Ansprüche 1 bis 6, **dadurch gekennzeichnet, dass** der mehrteilige Fotodetektor (DET) eine Zeile fotoempfindlicher Elemente (LCMOS) oder ein bildgebendes Spektrometer (IS) ist, wobei ein zusätzliches zylindrisches Optikelement zwischen der Lichtquelle (LS) und dem Vorrichtung (D1) vorkommt, und eine Strahlabtasteinrichtung im Objektarm des Interferometers zwischen dem mindestens einen Strahlteiler (BS) und dem optischen System U1 vorkommt, wobei eine Strahlabtasteinrichtung beispielsweise ein galvanometrischer Scanner, der so konfiguriert ist, dass er den Strahl in eine Richtung ablenkt (ID-GS), ist.

8. Vorrichtung nach einem der vorangehenden Ansprüche 1 bis 7, **dadurch gekennzeichnet, dass** der mehrteilige Fotodetektor (DET) eine eindimensionale Kamera (LCMOS) oder eine zweidimensionale Kamera (CMOS) ist und die Lichtquelle (LS) eine Vorrichtung, die einen Sweep von optischen Frequenzen oder Wellenlängen mit einer eine spektrale Bandbreite von mehr als 0,5 nm abdeckende Sweep-Spanne ermöglicht.

9. Vorrichtung nach Anspruch 7, **dadurch gekennzeichnet, dass** der mehrteilige Fotodetektor (DET) ein bildgebendes Spektrometer (IS) ist und die Lichtquelle (LS) eine Vorrichtung, die Erzeugung von räumlich kohärentem Licht (fs-LS) mit einem Spektrum ermöglicht, das sich über eine Bandbreite von mehr als 0,5 nm erstreckt, ist.

10. Ein Bildgebungsverfahren unter Mithilfe von optischer Kohärenztomographie mit Parallel-Fourier-Domänen-Detektion unter Verwendung einer spektral breitbandigen Lichtquelle (LS) und eines mehrteiligen Fotodetektors (DET) zum Erstellen einer dreidimensionalen Bildrekonstruktion der Struktur des Objekts (Ob), umfassend die Schritte:

   a) Aufspalten des Lichts durch einen Strahlteiler (BS) in einen Referenz- und Objektstrahl,
   b) dynamische Modulation der Lichtphase räumlich und zeitlich durch Verwendung der Vorrichtung (D1) und der Vorrichtung (D2), die zwischen der Lichtquelle und dem Strahlteiler angeordnet sind, **dadurch gekennzeichnet,**

**dass** die Vorrichtung (D1) für eine dynamische räumliche Lichtphasenmodulation über den Strahlquerschnitt sorgt und die Vorrichtung (D2) für eine unabhängige Steuerung der Winkelverteilung der Probenbeleuchtung sorgt, bevor der von der Lichtquelle (LS) emittierte Strahl durch den Strahlteiler (BS) in Referenz- und Objektstrahl aufgeteilt wird und die durch den mehrteiligen Fotodetektor (DET) erhaltenen Interferenzbilder aufwendig integriert werden.

11. Verfahren nach Anspruch 10, **dadurch gekennzeichnet, dass** sich die Phasenwerte im Strahlquerschnitt zufällig oder nach einer vorgegebenen Funktion ändern.

12. Verfahren nach Anspruch 10 oder 11, **dadurch gekennzeichnet, dass** eine komplexe Mittelwertbildung durch Integration auf einem mehrteiligen Fotodetektor (DET) bei gegebener Belichtungszeit durchgeführt wird.

13. Verfahren nach einem der vorangehenden Ansprüche 10 bis 12, **dadurch gekennzeichnet, dass** eine komplexe Mittelwertbildung durch numerische Summierung von Signalen komplexer Darstellungen von Interferenzbildern durchgeführt wird, die aus mehreren Messungen erhalten wurden.

14. Verfahren nach einem der vorhergehenden Ansprüche 10 bis 13, **dadurch gekennzeichnet, dass** das Bild des Strahlquerschnitts, in dem die räumliche Phasenmodulation erfolgt, in einer Objektebene gebildet wird.

15. Verfahren nach einem der vorhergehenden Ansprüche 10 bis 14, **dadurch gekennzeichnet, dass** das Ortsfrequenzspektrum, das dem Strahlquerschnitt entspricht, in dem die Ortsphasenmodulation auftritt, in einer Objektebene gebildet wird.

16. Verfahren nach einem der vorangehenden Ansprüche 10 bis 15, **dadurch gekennzeichnet, dass** die vom mehrteiligen Fotodetektor (DET) zeitlich sequentiell aufeinanderfolgend aufgenommenen interferometrischen Bilder den zeitlich unterschiedlichen der von der Breitbandlichtquelle (LS) generierten optischen Frequenzen entsprechen.

17. Verfahren nach einem der vorhergehenden Ansprüche 10 bis 16, **dadurch gekennzeichnet, dass** die interferometrischen Bilder in Form eines Satzes der von einem mehrteiligen Fotodetektor (DET) aufgenommenen Spektren aufgezeichnet werden.

18. Verfahren nach einem der vorhergehenden Ansprüche 10 bis 17, **dadurch gekennzeichnet, dass** der Vorgang der Bildrekonstruktion für unterschiedliche Beleuchtungswinkel des Objekts (Ob) wiederholt wird und anschließend die für unterschiedliche Beleuchtungswinkel erhaltenen Bildrekonstruktionen gemittelt werden, um eine endgültige Bildrekonstruktion der dreidimensionalen Struktur des Objekts (Ob) zu erhalten.

19. Verfahren nach einem der vorhergehenden Ansprüche 10 bis 18, **dadurch gekennzeichnet, dass** der das Objekt (Ob) beleuchtende Lichtstrahl zu einer Linie geformt wird und die räumliche Phasenmodulation entlang der durch den in eine Linie geformten Lichtstrahl definierten Richtung durchgeführt wird.

20. Abbildungsverfahren nach einem der vorhergehenden Ansprüche 10 bis 19, das in der Vorrichtung nach einem der vorhergehenden Ansprüche 1 bis 9 durchgeführt wird.

**Revendications**

1. Appareil d'imagerie d'objets par OCT à détection de domaine de Fourier parallèle, comprenant une source lumineuse (LS), un photodétecteur à éléments multiples (DET), un interféromètre contenant au moins un diviseur de faisceau (BS), un miroir de référence (M1), deux systèmes optiques U1, U2 et un système optique (U3) formant une image sur le photodétecteur multiélément (DET), **caractérisé en ce qu'**un appareil (D1) pour faire varier spatialement la modulation de la phase de la lumière sur la section transversale du faisceau et un appareil (D2) pour le contrôle indépendant de la distribution angulaire de l'éclairage de l'échantillon sont placés avant au moins un diviseur de faisceau (BS), configuré pour diviser le faisceau émis par la source lumineuse en un faisceau de référence et un faisceau objet.

2. Appareil selon la revendication 1, **caractérisé en ce que** l'appareil (D1) introduisant une modulation spatialement variable de la phase de la lumière comprend un élément actif choisi dans le groupe comprenant un diffuseur tournant ou coulissant, un miroir comprenant un réseau bidimensionnel des éléments piézoélectriques ou à cristaux liquides ou une membrane vibrante permettant de réfléchir la lumière, de préférence sous la forme d'un miroir déformable (DM).

3. Appareil selon la revendication 1, **caractérisé en ce que** l'appareil (D2) pour changement des angles d'éclairage est un dispositif numérique à micromiroir DMD, un déflecteur acousto-optique, un déflecteur électro-optique, un modulateur spatial de lumière à cristaux liquides, un microsystème électromécanique (MEMS) catoptrique ou au moins un scanner résonant ou galvanométrique (GM) équipé d'au moins un miroir monté sur au moins un moteur galvanométrique.

4. Appareil selon l'une quelconque des revendications précédentes de 1 à 2, **caractérisé en ce que** l'appareil (D1), configuré pour moduler la phase lumineuse, et le dispositif (D2), configuré pour contrôler l'angle d'éclairage de l'échantillon, sont physiquement séparés et configurés pour fonctionner indépendamment.

5. Appareil selon l'une quelconque des revendications précédentes de 1 à 4, **caractérisé en ce que** le système optique U1 formant lumière sur l'objet (Ob) et le système optique U2 formant lumière sur le miroir de référence (M1) comprennent des éléments optiques similaires.

6. Appareil selon l'une quelconque des revendications précédentes de 1 à 5, **caractérisé en ce que** le photodétecteur à éléments multiples (DET) est une caméra unidimensionnelle (LCMOS), une caméra bidimensionnelle (CMOS), un réseau de photodiodes ou spectromètre imageur (IS) permettant l'enregistrement simultané de spectres en les cartographiant en différents points de l'objet (Ob).

7. Appareil selon l'une quelconque des revendications précédentes de 1 à 6, **caractérisé en ce que** le photodétecteur à éléments multiples (DET) est une ligne d'éléments photosensibles (LCMOS) ou un spectromètre imageur (IS), dans lequel une optique cylindrique supplémentaire est entre la source lumineuse (LS) et l'appareil (D1), et un dispositif de balayage de faisceau est dans le bras objet de l'interféromètre entre au moins un diviseur de faisceau (BS) et le système optique U1, le dispositif de balayage de faisceau étant par exemple un scanner galvanométrique configuré pour dévier le faisceau dans une direction (ID-GS).

8. Appareil selon l'une quelconque des revendications précédentes de 1 à 7, **caractérisé en ce que** le photodétecteur à éléments multiples (DET) est une caméra unidimensionnelle (LCMOS) ou une caméra bidimensionnelle (CMOS), la source lumineuse (LS) est un appareil permettant un balayage de fréquences optiques ou de longueurs d'onde avec une plage de balayage couvrant une bande passante spectrale supérieure à 0,5 nm.

9. Appareil selon la revendication 7, **caractérisé en ce que** le photodétecteur à éléments multiples (DET) est un spectromètre imageant (IS), la source de lumière (LS) est un dispositif permettant de générer de la lumière spatialement cohérente (fs-LS) avec un spectre étendu sur une bande passante supérieure à 0,5 nm.

10. Une méthode d'imagerie utilisant la tomographie par cohérence optique avec détection de domaine de Fourier parallèle à l'aide d'une source de lumière à large bande spectrale (LS) et d'un photodétecteur à éléments multiples (DET) utilisé pour créer une reconstruction d'image tridimensionnelle de la structure de l'objet (Ob), comprenant les étapes de :

   a) répartition lumineuse par un diviseur de faisceau (BS) en un faisceau de référence et un faisceau objet,
   b) modulation dynamique spatiale et temporelle de la phase lumineuse à l'aide de l'appareil (D1) et de l'appareil (D2) placés entre la source lumineuse et le diviseur de faisceau **caractérisé en ce que** l'appareil (D1) assure une modulation spatiale dynamique de la phase lumineuse sur la section transversale du faisceau et l'appareil (D2) assure une commande indépendante de la distribution angulaire de l'éclairage de l'échantillon avant que le faisceau émis par la source lumineuse (LS) ne soit divisé en faisceaux de référence et objet par le diviseur de faisceau (BS), et les images d'interférence obtenues par le photodétecteur à éléments multiples (DET) sont intégrés de manière complexe.

11. Procédé selon la revendication 10, **caractérisé en ce que** les valeurs de phase dans la section transversale du faisceau évoluent de manière aléatoire ou selon une fonction prédéterminée.

12. Procédé selon la revendication 10 ou 11, **caractérisé en ce que** le moyennage complexe est effectué par intégration sur un photodétecteur à éléments multiples (DET) à un temps de pose donné.

13. Procédé selon l'une quelconque des revendications précédentes de 10 à 12, **caractérisé en ce que** le moyennage complexe est effectuée par sommation numérique de représentations complexes d'images d'interférence obtenues à partir de mesures multiples.

14. Procédé selon l'une quelconque des revendications précédentes de 10 à 13, **caractérisé en ce que** l'image de la section transversale du faisceau, dans laquelle a lieu la modulation de phase spatiale, est formée dans un plan objet.

15. Procédé selon l'une quelconque des revendications précédentes de 10 à 14, **caractérisé en ce que** le spectre de fréquences spatiales correspondant à la section transversale du faisceau dans laquelle se produit la modulation de phase spatiale est formé dans un plan objet.

16. Procédé selon l'une quelconque des revendications précédentes de 10 à 15, **caractérisé en ce que** les images interférométriques successives enregistrées séquentiellement dans le temps par le photodétecteur à éléments multiples (DET) correspondent aux différentes fréquences optiques générées dans le temps par une source lumineuse à large bande (LS).

17. Procédé selon l'une quelconque des revendications précédentes de 10 à 16, **caractérisé en ce que** les images interférométriques sont enregistrées sous la forme d'un ensemble de spectres enregistrés par le photodétecteur à éléments multiples (DET).

18. Procédé selon l'une quelconque des revendications précédentes de 10 à 17, **caractérisé en ce que** le processus de reconstruction d'image est répété pour différents angles d'éclairage de l'objet (Ob) et puis les reconstructions des images obtenues pour différents angles d'éclairage sont moyennées à obtenir une image finale de reconstruction de la structure tridimensionnelle de l'objet (Ob).

19. Procédé selon l'une quelconque des revendications précédentes de 10 à 18, **caractérisé en ce que** le faisceau lumineux éclairant l'objet (Ob) est conformé en une ligne et la modulation de phase spatiale est effectuée selon la direction définie par le faisceau lumineux formé dans une ligne.

20. Procédé d'imagerie selon l'une quelconque des revendications précédentes de 10 à 19 mis en œuvre dans l'appareil selon l'une quelconque des revendications précédentes de 1 à 9.

**Fig. 1**

**Fig. 2**

**Fig. 3**

**Fig. 4**

**Fig. 5**

**Fig. 6**

Fig. 7

Fig.8

**Fig. 9**

**Fig. 10**

**Fig. 11**

**1. Fd-FF-OCT + D2**

**2. Fd-FF-OCT + D1+D2**

**Fig. 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004088361 A
- US 2011134436 A1
- US 20140022554 A1
- US 20160299060 A1

**Non-patent literature cited in the description**

- **B. GRAJCIAR et al.** Parallel Fourier domain Optical Coherence Tomography for in vivo measurement of the human eye. *Optics Express,* 2005, vol. 13, 1131-1137
- **FECHTIG, D. J. et al.** Line-field parallel swept source MHz OCT for structural and functional retinal imaging. *Biomed. Opt. Express,* 2015, vol. 6, 716
- **HILLMANN, D. et al.** In vivo optical imaging of physiological responses to photostimulation in human photoreceptors. *Proc. Natl. Acad. Sci,* 2016, vol. 113, 13138-13143
- **BOCCARA, C. ; HARMS, F. ; LATRIVE, A.** Full-field OCT: a non-invasive tool for diagnosis and tissue selection. *SPIE Newsroom,* 2013, 2-5
- **THOUVENIN, O. ; APELIAN, C. ; NAHAS, A. ; FINK, M. ; BOCCARA, C.** Full-Field Optical Coherence Tomography as a Diagnosis Tool: Recent Progress with Multimodal Imaging. *Appl. Sci.,* 2017, vol. 7, 236
- **PENG XIAO ; MATHIAS FINK ; A. CLAUDE BOCCARA.** Full-field spatially incoherent illumination interferometry: a spatial resolution almost insensitive to aberrations. *Opt. Lett.,* 2016, vol. 41, 3920-3923
- **SCHWIDER, J. et al.** *Digital wave-front measuring interferometry: some systematic error sources,* 1983, vol. 22, 3421
- **CHOI, Y. ; YANG, TD ; LEE, KJ ; CHOI, W.** Full-field and single-shot quantitative phase microscopy using dynamic speckle illumination. *Opt. Lett.,* 2011, vol. 36, 2465
- **CHOI Y. et al.** Dynamic speckle illumination wide-field reflection phase microscopy. *Opt. Lett.,* 2014, vol. 39, 6062-6065
- **DESJARDINS, E. et al.** Angle-resolved Optical Coherence Tomography with sequential angular selectivity for speckle reduction. *Opt. Express,* 2007, vol. 15, 6200-6209
- **GRAJCAR et al.** Parallel Fourier domain optical coherence tomography for in vivo measurement of the human eye. *OPTICS EXPRESS,* 21 February 2005, vol. 13 (4), 1131